# Europäisches Patentamt

# European Patent Office

# Office européen des brevets

(19)

(11) Veröffentlichungsnummer: **0 318 642 B1**

(12) **EUROPÄISCHE PATENTSCHRIFT**

(45) Veröffentlichungstag der Patentschrift: **16.06.93**

(51) Int. Cl.5: **C07C 53/126**, C07C 51/41

(21) Anmeldenummer: **88113234.4**

(22) Anmeldetag: **16.08.88**

(54) **Aluminium-Magnesium-Hydroxi-Fettsäure-Verbindung enthaltende Gelzusammensetzumg.**

(30) Priorität: **25.09.87 DE 3732265**

(43) Veröffentlichungstag der Anmeldung:
**07.06.89 Patentblatt 89/23**

(45) Bekanntmachung des Hinweises auf die Patenterteilung:
**16.06.93 Patentblatt 93/24**

(84) Benannte Vertragsstaaten:
**AT BE CH DE ES FR GB GR IT LI LU NL SE**

(56) Entgegenhaltungen:
**EP-A- 0 308 646**
**US-A- 3 056 819**

(73) Patentinhaber: **Giulini Chemie GmbH**
**Giulinistrasse 2 Postfach 150 480**
**W-6700 Ludwigshafen/Rhein(DE)**

(72) Erfinder: **Martin, Roland, Dr., Dipl.-Ing.**
**Kastanienweg 21**
**W-6940 Weinheim(DE)**
Erfinder: **Schanz, Klaus, Dr., Dipl.-Chem.**
**In der Zeil 5**
**W-6701 Dannstadt-Schauernheim(DE)**
Erfinder: **Kaufmann, Bruno**
**Ormsheimerhof 16**
**W-6710 Frankenthal(DE)**

EP 0 318 642 B1

**Beschreibung**

Die vorliegende Erfindung bezieht sich auf eine neuartige Gelzusammensetzung, bestehend aus einer pulverförmigen Aluminium-Magnesium-Verbindung mit Schichtstruktur, einem polarem Additiv und einer bei Raumtemperatur flüssigen, organischen, lipophilen Verbindung, sowie auf seine Herstellung und Verwendung in der Kosmetik als rheologisches Additiv und Antiabsetzmittel.

Gute Anwendbarkeit, Stabilität und Reproduzierbarkeit bei der Herstellung von kosmetischen Präparaten hängen in erster Linie von den rheologischen Eigenschaften ihrer Bestandteile ab. In den meisten kosmetischen Präparaten sind Gele enthalten, die eine eigene Rheologie besitzen und damit auf die Eigenschaften der Fertigprodukte entscheidend Einfluß nehmen. Eine wichtige Eigenschaft der kosmetischen Gele ist die Thixotropie. Hier tritt bei einer Erhöhung der Scherkräftebehandlung eine deutliche Erniedrigung der Viskosität auf, d. h. ein scheinbar fester Stoff wird flüssig für eine bestimmte Zeit, denn es bildet sich langsam die ursprüngliche feste Gelstruktur wieder aus, wenn die Scherkräftebehandlung nachlässt. Einen großen Nutzen hat dieser Effekt in kosmetischen Präparaten wie z. B. Antiperspirantien, Cremes, Nagellacken aber auch in Anstrichfarben, Tinten und Seifen.

Kosmetische Gele bestehen im allgemeinen aus einem quellfähigen Tonmineral, das unter Aufnahme von Flüssigkeit ein Gel von hoher Viskosität bildet.

Bei der Gelbildung wird die Schichtstruktur des Tonminerals durch organische Flüssigkeiten aufgeweitet. Für die Aufnahme von organischen Flüssigkeiten wie z. B. Öle, Fette and Wachse muß das Tonmineral entsprechend gebaut sein, d. h. es muß organophil-oder lipophil sein oder dementsprechend chemisch verändert werden.

Die bekannten Gelzusammensetzungen enthalten Tonmineralien mit Schichtstruktur, z. B. aus der Reihe der Bentonite oder Montmorillonite deren Zusammensetzung der Formel entspricht:

$$(X,Y)_{2-3}(Si, Al)_4 O_{10}(OH)_2 Z_{1/3} \; nH_2O$$

in welcher X = Al, Fe(3), Mn(3) oder Cr(3); Y = Mg, Fe(2), Mn(2), Ni, Zn oder Li und Z = K, Na oder Ca ist.

Ein solches Silikat zeigt eine starke Hydrophilie und kann unter Absorbierung einer großen Wassermenge zwischen seinen Gitterebenen stark quellen und wässrige Gele mit hoher Viskosität bilden.

Weiterhin ist bekannt, daß man durch Ionenaustausch aus solchen hydrophilen Substanzen ein organophiles oder lipophiles Material herstellen kann. Dabei werden z. B. Na+ Ionen gegen positive quarternäre Ammoniumionen mit langkettigen, organischen Resten ausgetauscht (siehe dazu Jordan, J. W., Jour. Phys. and Colloid Chem. 53, 294, (1949) und Jordan J.W. und Mitarbeiter, Kolloid Z., 137, 40 (1954) oder Europäische Patentanmeldung 0 204 240 und DE OS 31 45 449 etc. Auf diese Weise hergestellte Materialien nennt man "organisch modifiziert". Diese organisch modifizierten Tonmineralien zeigen in Ölen, Fetten und Wachsen eine gute Quellwirkung und bilden mit ihnen unter Anwendung von mechanischer Energie, geeigneter Additive und bei geeigneter Temperatur viskose Gele. Es ist weiterhin eine Gelzusammensetzung bekannt, die ein organisch modifiziertes oder unmodifiziertes Tonmineral der Montmorillonitreihe, ein oberflächenaktives Mittel sowie ein organisches Lösungsmittel enthält (Grundstoffe und Verfahren der Arzneimittelzubereitung, 1960, Seite 715 v. F. Gstirner). Die Gelierfähigkeit dieses Montmorillonits ist jedoch sehr gering, so daß große Mengen des teuren Montmorillonits eingesetzt werden müssen, wobei dennoch Pigmente, die zugesetzt wurden, sehr leicht ausfielen und die gewünschte Viskosität der Gelzubereitung nicht ohne weiteres zu erhalten war.

Andere Gelzusammensetzungen enthalten 10 % organisch modifizierte Montmorillonite, 86,7 % Mineralöl und 3,3 % Netzmittel, welches auch als polares Additiv bezeichnet wird (H.P. Fiedler, Lexikon der Hilfsstoffe, EDITIO Cantor Aulendorf, S. 167).

Ein besonderer Nachteil der bekannten Gelzubereitungen besteht darin, daß durch die hohen prozentualen Gehalte der organisch polaren Additive wie Methanol, Ethanol, Aceton etc. und der durch den Ionentausch eingebrachten quarternären Ammoniumsalze hautreizende Wirkung haben und evtl. sogar Allergien auslösen können. Außerdem wird durch die Verunreinigungen der Montmorillonit-Minerale und durch die verschiedenen organischen Zusätze eine gelbe bis braune Farbe und ein unangenehmer Geruch nach den Netzmitteln erhalten. Gerade in der Kosmetik wird dies als besonders störend und unästhetisch empfunden.

Die Aufgabe der vorliegenden Erfindung bestand also darin, neue Gelzusammensetzungen zu finden, in denen quellfähige, gelbindende Verbindungen mit Schichtstruktur enthalten sind und diese Verbindungen die eingangs erwähnten Nachteile der bekannten Organotone nicht aufweisen. Es bestand ferner die Aufgabe, die Menge an polarem Additiv, die in den bekannten Gelzusammmensetzungen enthalten sind, drastisch zu senken und geeignete organische, bei Raumtemperatur flüssige Verbindungen zu finden, in denen die Gelbildung und die Quellung der Aluminium-Magnesium-Hydroxi-Verbindung optimal gewährleistet ist.

Die Aufgabe wird mit einer Gelzusammensetzug gemäß Anspruch 1 gelöst. Mit dieser Gelzusammensetzung können die eingangs erwähnten Nachteile beseitigt werden.

Die neuen Aluminium-Magnesium-Hydroxi-Verbindungen weisen eine Schichtstruktur auf und zeigen in organischen Flüssigkeiten sehr gute Gelbildung. Die Zusammensetzung und die Herstellung dieser Al/Mg-Hydroxide sind in einer noch nicht veröffentlichten Anmeldung beschrieben.

Gemäß der älteren Anmeldung haben die Verbindungen die allgemeine Formel

$$Al_x Mg_y (OH)_{35-z} R_z \ nH_2O$$

in der R der Rest $RCOO^-$ einer Monocarbonsäure mit 2 bis 22 Kohlenstoffatomen darstellt und die Indices x, y und z folgende Bedingungen erfüllen:

$3 \leq x \leq 9$
$4 \leq y \leq 13$
$3 \leq z \leq 5$
und $3x + 2y = 35$

Verbindungen, in denen $x = 5$, $y = 10$ und $z = 4$ ist, sind besonders geeignet. Als Monocarbonsäuren werden zweckmäßigerweise in die neuen Verbindungen technische Gemische von aliphatischen Monocarbonsäuren mit 16 bis 18 Kohlenstoffatomen eingearbeitet.

Die Herstellung der Verbindungen erfolgt durch Umsetzung der wässrigen Suspension einer Verbindung der Formel

$$Al_x Mg_y (OH)_{35-z} (SO_4)_{z/2} \ nH_2O$$

in der x, y die eben angegebene Bedeutung haben und für z gilt $3 < z < 5$, wobei $3x + 2y = 35$ ist, mit der wässrigen Suspension eines Alkalisalzes einer Monocarbonsäure, wobei der $RCOO^-$ Rest 2 bis 22 Kohlenstoffatome enthält, unter Rühren bei Temperaturen zwischen 20 C bis 100 C, bevorzugt zwischen 20 C und 60 C. Vorzugsweise arbeitet man unter Scherkräfteeinwirkung auf die wässrigen Suspensionen. Unter diesen Verfahrensbedingungen ist die Umsetzung in vielen Fällen nach 2 Stunden vollständig.

Die Abtrennung des Reaktionsproduktes aus der wässrigen Suspension kann nach einem der bekannten Verfahren erfolgen, bevorzugt jedoch durch Filtration. Der Filterkuchen muß mit Wasser zur Entfernung des anhaftenden Alkalisulfates so lange gewaschen werden, bis im Waschwasser mit Bariumchlorid kein $SO_4^{2-}$ nachweisbar ist. Die Trocknung des Filterkuchens wird bei Temperaturen zwischen 60 bis 130 C, bevorzugt aber bei 80 bis 110 C, z.B. in einem Hordentrockner vorgenommen. Andere Trockenvorrichtungen sind ebenfalls anwendbar.

Bei einer anderen Trocknungsvariante wird der sulfatfreie Filterkuchen in Wasser resuspendiert und sprühgetrocknet, wobei die Eintrittstemperatur $T_E = 250$ bis $350$ C, bevorzugt 270 bis 300 C, und die Austrittstemperatur $T_A = 80$ C bis 130 C, bevorzugt 90 bis 110 C, beträgt.

Nach anderen Verfahrensvarianten wird zur wässrigen Suspension der Verbindung

$$Al_x Mg_y (OH)_{35-z} (SO_4)_{z/2} \ nH_2O$$

das Alkalisalz einer Monocarbonsäure in fester Form zugesetzt, wobei alle anderen Verfahrensmerkmale gleich bleiben.

Die bei dem Verfahren als Ausgangsstoff eingesetzten Al, Mg-Verbindungen sind aus dem Stand der Technik bereits bekannt, z.B. aus der DE 34 08 463 C2. Die Monocarbonsäuren sind im Handel erhältliche Verbindungen. Die Herstellung der Alkalisalze kann gemäß Beispielen erfolgen.

Die Verbindungen sind feste, weiße und geruchslose, kristalline Stoffe. Die Charakterisierung ihrer Struktur geschieht mit Hilfe der Röntgendiffrakometrie und der Rasterelektronenmikroskopie. Durch die Röntgendiffraktometer-Aufnahmen konnte bewiesen werden, daß die Verbindungen kristallin sind. Die Schichten- oder Lamellenstruktur geht aus der beiliegenden REM-Aufnahme hervor. Die Abbildung 1 zeigt die REM-Aufnahme des Produktes aus Beispiel 10.

Im Vergleich mit einem organisch modifizierten Hectorit oder Natrium-Bentonit, die beide im Handel erhältlich sind, zeigen die Verbindungen gemäß der unveröffentlichten Anmeldung einen deutlich höheren Weißgrad.

Der Weißgrad ist ein Maß für die Farbe der Substanzen und wird z.B. mit dem Farbmeßgerät Tricolor LFM 3 von Dr. Lange gegen einen Emaille-Weißstandard gemessen. In der Tabelle 1 ist der Weißgrad der Produkte aus den nachstehenden Beispielen 6 - 17, sowie der Weißgrad von zwei Handelsprodukten angegeben. Aus dieser Tabelle geht es deutlich hervor, daß die neuen Verbindungen einen deutlich höheren Weißgrad besitzen, also fast weiß sind, während die Handelsprodukte gefärbt sind.

Tabelle 1

| Vergleich der Weißgrade | |
| --- | --- |
| Produkt aus Beispiel 6: | 98,1 |
| Produkt aus Beispiel 7: | 98,0 |
| Produkt aus Beispiel 8: | 98,1 |
| Produkt aus Beispiel 9: | 98,2 |
| Produkt aus Beispiel 10: | 97,9 |
| Produkt aus Beispiel 11: | 98,4 |
| Produkt aus Beispiel 12: | 98,3 |
| Produkt aus Beispiel 13: | 98,2 |
| Produkt aus Beispiel 14: | 98,1 |
| Produkt aus Beispiel 15: | 98,3 |
| Produkt aus Beispiel 16: | 98,4 |
| Produkt aus Beispiel 17: | 98,1 |
| Natrium-Bentonit | 91,3 |
| Org.modif. Hectorit | 91,8 |

Die Wirksamkeit des neuen Produktes als Antiabsetzmittel schon in Konzentration von 2 % zeigen die nachstehenden Versuche:

Es wurden Formulierungen, wie in Tabelle 2 beschrieben, hergestellt und die Absetzkurve über eine Trübungsmessung mit Hilfe eines Eppendorf-Photometers bestimmt:

In einem 300 ml Becherglas wird die Extinktion des Lösungsmittels Paraffinöl auf 0 eingestellt, d.h. 100 % Durchlässigkeit. In das gleiche Becherglas werden nun die Produkte aus den einzelnen Beispielen im Vergleich mit im Handel erhältlichem Na-Bentonit und einem org. modif. Hectorit in 2 %iger Konzentration in Paraffinöl gegeben, durch Rühren homogen suspendiert und danach 3 Min. lang mit 100 U/min. gerührt. Der Rührer wird abgestellt und die Abnahme der Extinktion über einen Schreiber verfolgt. Der Extinktionswert, den man direkt nach dem Abstellen des Rührers erhält, wird mit 0 % Durchlässigkeit angenommen.

Aus der Tabelle 2 kann man leicht erkennen, daß die hergestellten Produkte sich wesentlich schwerer als die Vergleichsprodukte absetzen, was ein Vorteil bei Pigment-Farben ist.

Tabelle 2

| Absetzversuche (% Durchlässigkeit) | | | | | |
|---|---|---|---|---|---|
| Produkt aus Beispiel | 1 h | 2 h | 3 h | 6 h | 8 h |
| 6 | 0,2 | 0,4 | 1,0 | 11,9 | 21,0 |
| 7 | 0,8 | 1,2 | 1,4 | 4,7 | 9,0 |
| 8 | 1,0 | 2,0 | 2,8 | 13,6 | 23,5 |
| 9 | 0,8 | 0,8 | 1,2 | 4,7 | 8,5 |
| 10 | 0,8 | 0,8 | 0,8 | 3,5 | 7,0 |
| 11 | 0,8 | 0,8 | 1,2 | 2,5 | 5,8 |
| 12 | 0,4 | 0,4 | 0,4 | 1,6 | 5,5 |
| 13 | 0,2 | 0,2 | 0,2 | 1,5 | 5,3 |
| 14 | 0,2 | 0,2 | 0,3 | 1,5 | 5,0 |
| 15 | 0,2 | 0,2 | 0,2 | 1,6 | 5,4 |
| 16 | 0,4 | 0,6 | 0,8 | 2,0 | 6,1 |
| 17 | 0,6 | 0,6 | 1,0 | 2,5 | 6,3 |
| Na-Bentonit: | 2,3 | 7,6 | 12,5 | 33,0 | 51,0 |
| Org.mod. Hectorit: | 2,0 | 6,5 | 9,0 | 22,2 | 33,0 |

Separat wird hierzu das Absetzvolumen nach verschiedenen Zeiten in einem 100 ml Meßzylinder bestimmt. Dazu wird die Formulierung 20mal vertikal und 20mal horizontal geschüttelt und dann stehen gelassen. Das Absetzvolumen gibt Auskunft über die leichte Dispergierbarkeit der organischen Produkte gem. unveröffentlichter Anmeldung in diesem Lösungsmittel unter geringen Scherkräften.

Im Unterschied zu den Absetzversuchen aus den Tabellen 2/3 wurden hier die Produkte nach der unveröffentlichten Anmeldung (2 %) mit einem, in diesem Lösungsmittel unlöslichen Stoff (z.B. Aluminiumchlorhydrat mit einer Korngröße von 90 % im Bereich zwischen 10 und 75 um) in 15 %iger Konzentration in dem jeweiligen Lösungsmittel suspendiert. Man kann auch erkennen, daß sich die suspendierten Produkte bei Zugabe der Substanzen aus den Beispielen 6 - 11 schwerer absetzen.

Tabelle 3

| Absetzvolumen (ml) in Siliconöl (Typ 345 von Dow Corning) | | | | | | | | | | |
|---|---|---|---|---|---|---|---|---|---|---|
| Produkt aus | 0,5 min | 1 min | 3 min | 7 min | 10 min | 20 min | 30 min | 60 min | 2 h | 14 h |
| Beispiel 6 | 99 | 98 | 98 | 91 | 90 | 80 | 75 | 65 | 53 | 23 |
| Beispiel 7 | 100 | 100 | 99 | 98 | 97 | 93 | 89 | 74 | 58 | 24 |
| Beispiel 8 | 100 | 100 | 98 | 96 | 95 | 90 | 85 | 72 | 56 | 24 |
| Beispiel 9 | 100 | 99 | 98 | 96 | 94 | 88 | 82 | 77 | 61 | 27 |
| Beispiel 10 | 100 | 99 | 98 | 96 | 94 | 89 | 81 | 70 | 54 | 27 |
| Beispiel 11 | 100 | 99 | 97 | 94 | 92 | 84 | 78 | 67 | 54 | 25 |
| Ohne Zusatz | 98 | 95 | 73 | 44 | 40 | 35 | 32 | 30 | 25 | 25 |
| Na-Bentonit: | 100 | 99 | 97 | 82 | 68 | 47 | 43 | 38 | 35 | 25 |
| Org.mod. Hecorit: | 100 | 99 | 97 | 92 | 91 | 82 | 72 | 62 | 50 | 27 |

EP 0 318 642 B1

Tabelle 4

| Absetzvolumen (ml) in Paraffinöl (Typ Pioneer 2660, hochviskos) von der Firma Hansen + Rosenthal | | | | | | | | | |
|---|---|---|---|---|---|---|---|---|---|
| Produkt aus | 10 min | 30 min | 1 h | 2 h | 3 h | 4 h | 8 h | 18 h | 24 h |
| Beispiel 6 | 100 | 98 | 97 | 91 | 87 | 82 | 59 | 45 | 43 |
| Beispiel 7 | 100 | 99 | 98 | 96 | 94 | 93 | 77 | 47 | 43 |
| Beispiel 8 | 100 | 99 | 98 | 94 | 92 | 89 | 71 | 48 | 43 |
| Beispiel 9 | 100 | 99 | 97 | 94 | 91 | 89 | 72 | 44 | 43 |
| Beispiel 10 | 100 | 99 | 98 | 94 | 92 | 90 | 73 | 45 | 43 |
| Beispiel 11 | 100 | 98 | 98 | 93 | 91 | 88 | 67 | 45 | 43 |
| Na-Bentonit: | 100 | 96 | 87 | 61 | 53 | 49 | 44 | 43 | 43 |
| org.mod. Hectorit: | 99 | 98 | 97 | 91 | 85 | 80 | 58 | 45 | 43 |
| ohne Zusatz: | 97 | 94 | 84 | 60 | 50 | 47 | 41 | 40 | 40 |

Anhand der nachstehenden Beispiele wird der Gegenstand der früheren Anmeldung näher erläutert.

Beispiel 1:

Herstellung von

$$Al_xMg_y(OH)_{35-z}(SO_4)_{z/2} \cdot nH_2O$$

In einem offenen Rührkessel werden 4 743 g Aluminiumhydroxid-Paste mit 12,73 Gew.% $Al_2O_3$ vorgelegt, mit 8 995 g Wasser verdünnt und danach 2 924,8 g Aluminiumsulfat-Lösung mit 21,54 % $SO_4$ und 4,21 % Al eingerührt. Man läßt über Nacht stehen, damit eventuell vorhandenes $CO_2$-Gas entweichen kann. Es wird 1 336,7 g MgO mit 60,3 % Mg-Gehalt unter Rühren hinzugeben, wobei eine leichte Erwärmung eintritt. Man läßt noch 2 Stunden weiterrühren und kann dann die Suspension zur weiteren Verarbeitung verwenden.
Analyse: 2,46 % Al, 4,47 % Mg, 3,5 % $SO_4$

Beispiel 2: Herstellung von Natriumcaprylat $C_7H_{15}COONa$

800 g Caprylsäure werden in 7 l Wasser suspendiert und unter Rühren auf 80 C erhitzt. Danach gibt man langsam eine Lösung von 221,8 g NaOH in 500 g Wasser zu und läßt auf Raumtemperatur abkühlen. Die wässrige Lösung dampft man langsam ein und trocknet den Rückstand bei 105 C im Trockenschrank.
Ausbeute: 877 g (95 % d.Th.) weißes Pulver

Beispiel 3: Herstellung von Natriummyristat $C_{13}H_{27}COONa$

800 g Myristinsäure werden in 3 l Wasser suspendiert und unter Rühren auf 80 C erhitzt. Danach gibt man langsam eine Lösung von 140,2 g NaOH in 350 ml Wasser zu und läßt auf Raumtemperatur abkühlen. Dabei fällt das Natriummyristat aus, welches über eine Nutsche abfiltriert wird. Es wird vorsichtig im Trockenschrank bis auf Gewichtskonstanz getrocknet.
Ausbeute: 820 g (89 % d.Th.) weißes Pulver

Beispiel 4: Herstellung von Natriumpalmitat $C_{15}H_{31}COONa$

800 g Palmitinsäure werden in 9 l Wasser suspendiert und unter Rühren auf 80 Grad C erwärmt. Danach gibt man eine Lösung von 124,8 g NaOH in 350 ml Wasser zu und läßt auf Raumtemperatur abkühlen. Man nutscht ab und trocknet den Rückstand bei 105 Grad C im Trockenschrank.
Ausbeute: 814 g (94 % d.Th.) weißes Pulver

Beispiel 5: Herstellung von Natriumbehenat $C_{21}H_{43}COONa$

700 g Behensäure werden in 9 000 ml Wasser suspendiert und auf 80 C erhitzt. Danach gibt man eine Lösung von 83 g NaOH in 350 ml dest. Wasser hinzu. Dabei fällt sofort das Natriumbehenat aus. Man läßt auf Raumtemperatur abkühlen und filtriert den Niederschlag über eine Nutsche ab. Mit je 3 x 200 ml Ethanol wird nachgewaschen und der Rückstand bei 65 C im Trockenschrank getrocknet.
Ausbeute: 708 g (95 % d.Th.) weißes Pulver

Beispiel 6: Herstellung von

$Al_5 Mg_{10} (OH)_{31} (CH_3 COO)_4$

119,6 g Na-acetat werden in 1 076 g Wasser mittels Rührer suspendiert und zu 2 000 g Al, Mg-hydroxisulfat-Suspension, wie in Beispiel 1 hergestellt, gegeben. Man erwärmt 3 Stunden auf 80 C, um die Reaktion zu vervollständigen, läßt abkühlen und filtriert dann das unlösliche Al, Mg-hydroxi-acetat ab. Mit Wasser wird so lange nachgewaschen, bis kein Sulfat mehr mit BaCl<t2 Lösung als $BaSO_4$ nachweisbar ist. Der Filterkuchen wird dann im Trockenschrank bei 105 C bis auf Gewichtskonstanz getrocknet.
Ausbeute: 395 g (95 % d. Th.)
Beschreibung: weißes, geruchloses, kristallines Pulver
Identifikation: 11,5 % Al i. Tr. (Theorie 11,8 %)
20,9 % Mg i. Tr. (Theorie 21,3 %)
8,3 % C i. Tr. (Theorie 8,4 %)

| Röntgenspektrum (Gerät: Philipps Automated X-Ray Powder Diffraktometer, System APD 15) zu Beispiel 6 | | | |
|---|---|---|---|
| Peak Nr. | 2 - Theta | d ( pm ) | I / I o |
| 1 | 19,870 | 4 46,45 | 82 |
| 2 | 34,170 | 2 62,18 | 56 |
| | 34,484 | 2 59,86 | 74 |
| | 35,669 | 2 55,07 | 100 |
| | 35,669 | 2 51,50 | 77 |
| 3 | 41,400 | 2 17,00 | |
| 4 | 42,500 | 2 13,00 | |
| 5 | 48,200 | 1 89,00 | |
| 6 | 60,309 | 1 53,34 | 49 |
| | 60,526 | 1 52,84 | 66 |
| | 61,199 | 1 51,32 | 76 |
| | 61,633 | 1 50,35 | 60 |
| | 61,965 | 1 49,63 | 58 |

Beispiel 7: Herstellung von Al-Mg-hydroxi-caprylat

$Al_5 Mg_{10} (OH)_{31} (C_7 H_{15} COO)_4$

242,3 g Na-caprylat (aus Beispiel 2) werden in 2 181 g Wasser mittels Rühren suspendiert und zu 2 000 g Al,Mg-hydroxi-sulfat-Suspension, wie in Beispiel 1 hergestellt, gegeben. Man erwärmt 1 Stunde auf 60 C, um die Reaktion zu vervollständigen, läßt abkühlen und filtriert dann das unlösliche Al, Mg-hydroxi-caprylat ab. Mit Wasser wird so lange nachgewaschen, bis kein Sulfat mehr mit $BaCl_2$ -Lösung als $BaSO_4$ nachweisbar ist. Der Filterkuchen wird dann im Trockenschrank bei 105 C bis auf Gewichtskonstanz getrocknet.
Ausbeute: 517 g (96 % d. Th.)
Beschreibung: weißes, geruchloses, kristallines Pulver
Identifikation: 9,0 % Al. i. Tr. (Theorie 9,1 %)
16,2 % Mg. i. Tr. (Theorie 16,4 %)
25,0 % C i. Tr. (Theorie 26,0 %)

| Röntgenspektrum (Gerät: Philipps Automated X-Ray Powder  Diffraktometer, System APD 15) zu Beispiel 7 | | | |
|---|---|---|---|
| Peak Nr. | 2 - Theta | d ( pm ) | I / I o |
| 1 | 19,370 | 4 57,84 | 66 |
| 2 | 33,974 | 2 63,65 | 42 |
|  | 34,267 | 2 61,46 | 72 |
|  | 34,506 | 2 59,70 | 88 |
|  | 34,871 | 2 57,07 | 100 |
|  | 35,075 | 2 55,62 | 83 |
|  | 35,608 | 2 51,91 | 62 |
| 3 | 41,4 | 2 17, |  |
| 4 | 42,5 | 2 13, |  |
| 5 | 48,3 | 1 89, |  |
| 6 | 60,652 | 1 525,5 | 67 |
|  | 60,892 | 1 520,1 | 72 |
|  | 61,279 | 1 511,4 | 56 |
|  | 61,714 | 1 501,8 | 46 |

Beispiel 8: Herstellung von Al-Mg-hydroxi-myristat

$Al_5 Mg_{10} (OH)_{31} (C_{13} H_{27} COO)_4$

182,5 g Na-myristat (aus Beispiel 3) werden in 1 643 g Wasser mittels Rühren suspendiert und zu 1 000 g Al, Mg-hydroxi-sulfat-Suspension, wie in Beispiel 1 hergestellt, gegeben. Man erwärmt 1 Stunde auf 60 C, um die Reaktion zu vervollständigen, läßt abkühlen und filtriert dann das unlösliche Al, Mg-hydroxi-myristat ab. Mit Wasser wird so lange nachgewaschen, bis kein Sulfat mehr mit $BaCl_2$ -Lösung als $BaSO_4$ nachweisbar ist. Der Filterkuchen wird dann im Trockenschrank bei 105 C bis auf Gewichtskonstanz getrocknet.
Ausbeute: 321 g (97 % d. Th.)
Beschreibung: weißes, geruchloses, kristallines Pulver
Identifikation: 7,3 % Al i. Tr. (Theorie 7,4 %)
13,2 % Mg i. Tr. (Theorie 13,4 %)
36,3 % C i. Tr. (Theorie 37,0 %)

| Röntgenspektrum (Gerät: Philipps Automated X-Ray Powder Diffraktometer, System APD 15) zu Beispiel 8 | | | |
|---|---|---|---|
| Peak Nr. | 2 - Theta | d ( pm ) | I / I o |
| 1 | 20,909 | 4 24,48 | 100 |
|  | 21,340 | 4 16,00 | 91 |
|  | 21.560 | 4 11,82 | 72 |
| 2 | 33,916 | 2 64,08 | 35 |
|  | 34,089 | 2 62,78 | 49 |
|  | 34,471 | 2 59,95 | 70 |
|  | 35,069 | 2 55,66 | 68 |
|  | 35,524 | 2 52,49 | 51 |
| 3 | 41,4 | 2 17, |  |
| 4 | 42,5 | 2 13, |  |
| 5 | 48,2 | 1 89, |  |
| 6 | 60,318 | 1 53,31 | 28 |
|  | 60,534 | 1 52,82 | 47 |
|  | 61,649 | 1 50,32 | 42 |

Beispiel 9: Herstellung von Al-Mg-hydroxi-palmitat

$Al_5 Mg_{10} (OH)_{31} (C_{15} H_{31} COO)_4$

405,9 g Na-palmitat (aus Beispiel 4) werden in 3 653 g Wasser mittels Rühren suspendiert und zu 2 000 g Al, Mg-hydroxi-sulfat-Suspension, wie in Beispiel 1 hergestellt, gegeben. Man erwärmt 1 Stunde auf 60 grad C, um die Reaktion zu vervollständigen, läßt abkühlen und filtriert dann das unlösliche Al, Mg-hydroxi-palmitat ab. Mit Wasser wird so lange nachgewaschen, bis kein Sulfat mehr mit $BaCl_2$ - Lösung als $BaSO_4$ nachweisbar ist. Der Filterkuchen wird dann im Trockenschrank bei 105 C bis auf Gewichtskonstanz getrocknet.
Ausbeute: 660 g (94 % d. Th.)
Beschreibung: weißes, geruchloses, kristallines Pulver
Identifikation: 6,8 % Al i. Tr. (Theorie 7,0 %)
12,4 % Mg i. Tr. (Theorie 12,6 %)
39,4 % C i. Tr. (Theorie 39,9 %)

| Röntgenspektrum (Gerät: Philipps Automated X-Ray Powder Diffraktometer, System APD 15) zu Beispiel 9 | | | |
|---|---|---|---|
| Peak Nr. | 2 - Theta | d ( pm ) | I / I o |
| 1 | 19,702 | 4 50,21 | 59 |
|  | 21,323 | 4 16,35 | 100 |
| 2 | 31,792 | 2 81,23 | 30 |
| 3 | 34,305 | 2 61,17 | 48 |
|  | 34,615 | 2 58,91 | 76 |
|  | 35,169 | 2 54,95 | 64 |
| 4 | 41,400 | 2 17, |  |
| 5 | 42,5 | 2 13, |  |
| 6 | 48,3 | 1 89, |  |
| 7 | 60,700 | 1 52,44 | 49 |
|  | 61,138 | 1 51,45 | 32 |

Beispiel 10: Herstellung von Al-Mg-hydroxi-stearat

$Al_5 Mg_{10}(OH)_{31}(C_{17}H_{35}COO)_4$

446,8 g Na-stearat werden in 4 021 g Wasser mittels Rühren suspendiert und zu 2 000 g Al, Mg-hydroxi-sulfat-Suspension, wie in Beispiel 1 hergestellt, gegeben. Man erwärmt 1 Stunde auf 60 C um die Reaktion zu vervollständigen, läßt abkühlen und filtriert dann das unlösliche Al, Mg-hydroxi-stearat ab. Mit Wasser wird dann so lange nachgewaschen, bis kein Sulfat mehr mit $BaCl_2$ - Lösung als $BaSO_4$ nachweisbar ist. Der Filterkuchen wird dann im Trockenschrank bei 105 C bis auf Gewichtskonstanzgetrocknet

Ausbeute: 738 g (98 % d. Th.)

Beschreibung: weißes, geruchloses, kristallines Pulver

Identifikation: 6,5 % Al i. Tr. (Theorie 6,6 %)

11,7 % Mg i. Tr. (Theorie 11,9 %)

42,2 % C i. Tr. (Theorie 42,4 %)

Röntgenaufnahme zu Beispiel 10:

| Röntgenspektrum (Gerät: Philipps Automated X-Ray Powder  Diffraktometer, System APD 15) zu Beispiel 10 | | | |
|---|---|---|---|
| Peak Nr. | 2 - Theta | d ( pm ) | I / I o |
| 1 | 19,576 | 4 53,07 | 59 |
|  | 20,794 | 4 26,81 | 91 |
|  | 21,466 | 4 13,59 | 100 |
| 2 | 31,641 | 2 82,53 | 34 |
| 3 | 34,894 | 2 64,25 | 41 |
|  | 34,072 | 2 62,91 | 36 |
|  | 34,401 | 2 60,47 | 47 |
|  | 34,697 | 2 58,32 | 61 |
|  | 35,265 | 2 54,29 | 46 |
|  | 35,5114 | 2 52,56 | 44 |
|  | 35,693 | 2 51,33 | 39 |
|  | 35,873 | 2 50,11 | 34 |
| 4 | 41,4 | 2 17, |  |
| 5 | 42,5 | 2 13, |  |
| 6 | 48,163 | 1 88,77 | 30 |
| 7 | 60,399 | 1 53,13 | 26 |
|  | 60,765 | 1 52,29 | 37 |
|  | 60,974 | 1 51,82 | 35 |
|  | 61,306 | 1 51,08 | 35 |

Themoanalyse zu Beispiel 10:

Thermische
Differenzialanalyse

Thermogravimetrische
Analyse

545    380    320 206    °C

Beispiel 11: Herstellung von Al-Mg-Hydroxi-behenat

$Al_5 Mg_{10} (OH)_{31} (C_{21} H_{43} COO)_4$

528,6 g Na-behenat (aus Beispiel 5) werden in 4 758 g Wasser mittels Rühren suspensiert und zu 2 000 g Al, Mg-hydroxi-sulfat-Suspension, wie in Beispiel 1 hergestellt, gegeben. Man erwärmt 1 Stunde auf 60 C, um die Reaktion zu vervollständigen, läßt abkühlen und filtriert dann das unlösliche Al, Mg-hydroxi-behenat ab. Mit Wasser wird so lange nachgewaschen, bis kein Sulfat mehr mit BaCl$_2$ -Lösung als BaSO$_4$ nachweisbar ist. Der Filterkuchen wird dann im Trockenschrank bei 105 C bis auf Gewichtskonstanz getrocknet.

Ausbeute: 767 g (95 % d. Th.)

Beschreibung: weißes, geruchloses, kristallines Pulver

Identifikation: 5,8 % Al i. Tr. (Theorie 6,0 %)

10,5 % Mg i. Tr. (Theorie 10,7 %)

46,2 % C i. Tr. (Theorie 46,7 %)

| Röntgenspektrum (Gerät: Philipps Automated X-Ray Powder Diffraktometer, System APD 15) zu Beispiel 11 | | | |
|---|---|---|---|
| Peak Nr. | 2 - Theta | d ( pm ) | I / I o |
| 1 | 19,584 | 4 52,90 | 67 |
|  | 21,049 | 4 21,69 | 82 |
|  | 21,260 | 4 17,56 | 100 |
| 2 | 31,851 | 2 80,72 | 43 |
| 3 | 34,074 | 2 62,89 | 46 |
|  | 34,630 | 2 58,80 | 61 |
|  | 34,857 | 2 57,17 | 73 |
|  | 35,176 | 2 54,90 | 54 |
|  | 35,686 | 2 51,38 | 52 |
| 4 | 41,4 | 2 17, |  |
| 5 | 42,5 | 2 13, |  |
| 6 | 48,3 | 1 89, |  |
| 7 | 60,705 | 1 52,43 | 43 |
|  | 61,982 | 1 49,59 | 28 |

Beispiel 12: Herstellung von Al-Mg-hydroxi-stearat

Al$_3$Mg$_{13}$(OH)$_{31}$(C$_{17}$H$_{35}$COO)$_4$

In einem offenen Rührkessel werden 578,2 g Aluminiumhydroxid-Paste mit 12,73 % Al$_2$O$_3$ vorgelegt, mit 3 151,4 g Wasser verdünnt und danach 796 g Aluminiumsulfat-Lösung welche 4,22 % Al und 21,62 % SO$_4$ enthält, eingerührt. Man läßt über Nacht stehen, damit eventuell vorhandenes CO$_2$ -Gas entweichen kann und fügt dann 474 g MgO (im Handel erhältlich) mit 99 % MgO-Gehalt unter Rühren hinzu. Dabei tritt eine leichte Erwärmung ein.

Analyse der Suspension: 1,40 % Al, 5,60 % Mg, 3,46 % SO$_4$

Zu 4 469 g der obigen Suspension fügt man unter Rühren 986 g Na-stearat in 7 000 g Wasser suspendiert hinzu. Man erwärmt eine Stunde auf 60 C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 270 C und die Austrittstemperatur 100 C.

Ausbeite: 1 606 g (97 % d. Th.)

Beschreibung: weißes, geruchloses, kristallines Pulver

Identifikation: 3,7 % Al i. Tr. (Theorie 3,9 %)

15,3 % Mg i. Tr. (Theorie 15,4 %)

40,8 % C i. Tr. (Theorie 41,5 %)

Beispiel 13: Herstellung von Al-Mg-hydroxi-stearat

Al$_5$Mg$_{10}$(OH)$_{31}$(C$_{17}$H$_{35}$COO)$_4$

In einem offenen Rührkessel werden 1 581 g Aluminiumhydroxid-Paste mit 12,3 % Al$_2$O$_3$ vorgelegt, mit 3

000 g Wasser verdünnt und danach 975 g Aluminiumsulfat-Lösung (festes Aluminiumsulfat im Handel erhältlich), welche 4,21 % Al und 21,54 % $SO_4$ enthält, eingerührt. Man läßt über Nacht stehen, damit eventuell vorhandenes $CO_2$-Gas entweichen kann und fügt dann 446 g MgO mit 99% MgO-Gehalt unter Rühren hinzu. Dabei tritt eine leichte Erwärmung ein.

Analyse der Suspension: 2,41 % Al, 4,40 % Mg, 3,45 % $SO_4$

Zu 4 470 g der obigen Suspension fügt man unter Rühren 983 g Natriumstearat in 7 000 g Wasser suspendiert hinzu. Man erwärmt eine Stunde auf 60 C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 275 C und die Austrittstemperatur 100 C.

Ausbeute: 1 556 g (95 % d. Th.)

Beschreibung: weißes, geruchloses, kristallines Pulver

Identifikation: 6,5 % Al i. Tr. (Theorie 6,6 %)

11,7 % Mg i. Tr. (Theorie 11,9 %)

42,0 % C i.Tr. (Theorie 42,4 %)

Dichte: 1,19 g/ml

Beispiel 14: Herstellung von Al-Mg-hydroxi-stearat

$Al_7 Mg_7 (OH)_{31} (C_{17} H_{35} COO)_4$

In einem offenen Rührkessel werden 2 086 g Aluminiumhydroxid-Paste mit 12,73 % $Al_2 O_3$ vorgelegt, mit 1 825 g Wasser verdünnt und danach 824 g Aluminiumsulfat-Lösung, welche 4,22 % Al und 21,62 % $SO_4$ enthält, eingerührt. Man läßt über Nacht stehen, damit eventuell vorhandenes $CO_2$-Gas entweichen kann und fügt dann 264 g MgO mit 99 % MgO-Gehalt unter Rühren hinzu. Dabei tritt eine leichte Erwärmung ein.

Analyse der Suspension: 3,4 % Al, 3,2 % Mg, 3,7 % $SO_4$

Zu 4 469 g der obigen Suspension fügt man unter Rühren 1 054 g Natrium-stearat in 7 000 g Wasser suspendiert hinzu. Man erwärmt eine Stunde auf 60 C, läßt abkühlen und filtriert dann das unlösliche Al-Mg-hydroxi-stearat ab. Mit Wasser wird so lange nachgewaschen, bis kein Sulfat mehr nachweisbar ist. Der Filterkuchen wird wieder in 10 kg Wasser suspendieret und sprühgetrocknet. Die Eintrittstemperatur beträgt 270 C und die Austrittstemperatur 100 C.

Ausbeute: 1 668 g (96 % d. Th.)

Beschreibung: weißes, geruchloses, kristallines Pulver

Identifikation: 9,2 % Al i. Tr. (Theorie 9,3 %)

8,1 % Mg i. Tr. (Theorie 8,4 %)

41,9 % C i. Tr. (Theorie 42,3 %)

Beispiel 15: Herstellung von Al-Mg-hydroxi-stearat

$Al_9 Mg_4 (OH)_{31} (C_{17} H_{35} COO)_4$

In einem offenen Rührkessel werden 2 881 g Aluminiumhydroxid-Paste mit 12,73 % $Al_2 O_3$ vorgelegt, mit 1 126 g Wasser verdünnt und danach 839 g Aluminiumsulfat-Lösung, welche 4,22 % Al und 21,62 % $SO_4$ enthält, eingerührt. Man läßt über Nacht stehen, damit eventuell vorhandenes $CO_2$-Gas entweichen kann und fügt dann 154 g MgO (im Handel erhältlich) mit 99 % MgO-Gehalt unter Rühren hinzu. Dabei tritt eine leichte Erwärmung ein.

Analyse der Suspension: 4,32 % Al, 4,87 % Mg, 3,85 % $SO_4$

Zu 4 469 g der obigen Suspension fügt man unter Rühren 1 098 g Natrium-stearat in 7 000 g Wasser suspendiert hinzu. Man erwärmt eine Stunde auf 60 C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 270 C und die Austrittstemperatur 100 C.

Ausbeute: 1 685 (94 % d. Th.)

Beschreibung: weißes, geruchloses, kristallines Pulver

Identifikation: 12,0 % Al i. Tr. (Theorie 12,1 %)

4,7 g Mg i. Tr. (Theorie 4,9 %)

42,4 % C i. Tr. (Theorie 42,7 %)

Beispiel 16: Herstellung von Al-Mg-hydroxi-stearat

$Al_5 Mg_{10} (OH)_{31} (C_{17} H_{35} COO)_4$

In einem 200 l-Rührkessel werden 11,1 kg Aluminiumhydroxid-Paste mit 12,3 % $Al_2O_3$ vorgelegt, mit 30 kg Wasser verdünnt und danach 6,8 kg Aluminiumsulfat-Lösung, welche 4,2 % Al und 21,5 % $SO_4$ enthält, eingerührt. Man rührt 3 Stunden nach und fügt dann 3,1 kg MgO mit 99 % MgO-Gehalt hinzu. Dabei tritt eine leichte Erwärmung ein. Nach weiteren 3 Stunden Rühren gibt man 7,2 kg Natrium-stearat und 49 kg Wasser hinzu. Man rührt noch 2 Stunden und behandelt dann die Suspension mit hohen Scherkräften, um eine homogene Paste zu erhalten. Nach weiterem Rühren (ca. 1 Stunde) filtriert man das unlösliche Al, Mg-hydroxi-stearat über eine Filterpresse ab. Mit Wasser wird so lange nachgewaschen, bis kein Sulfat mehr nachweisbar ist. Der Filterkuchen wird in 70 kg Wasser suspendiert und sprühgetrocknet. Die Eintrittstemperatur beträgt 280 C und die ausgangstemperatur 90 C.
Ausbeute: 10,5 kg (92% der Theorie)
Beschreibung: weißes, geruchloses, kristallines Pulver
Identifikation: 6,5 % Al i. Tr. (Theorie 6,6 %)
11,8 % Mg i. Tr. (Theorie 11,9 %)
42,1 % C i. Tr. (Theorie 42,4 %)

Beispiel 17: Herstellung von Al-Mg-hydroxi-palmitat-stearat

$Al_5 Mg_{10} (OH)_{31} (C_{15} H_{31} COO)(C_{17} H_{35} COO)_3$

101 g Na-palmitat (aus Beispiel 4) und 335 g Na-stearat werden in 3 930 g Wasser unter Rühren suspendiert und zu 2 000 g Al-Mg-hydroxisulfat-Suspension, wie in Beispiel 1 hergestellt, gegeben. Man homogenisiert mit einem Turrax und rührt 3 Stunden bei Raumptemperatur. Man filtriert ab und wäscht mit dest. Wasser sulfatfrei. Der Filterkuchen wird bei 95 C im Trockenschrank bis auf Gewichtskonstanz getrocknet.
Ausbeute: 693 g (95 % der Theorie)
Beschreibung: weißes, geruchloses, kristallines Pulver
Identifikation: 6,6 Al i. Tr. (Theorie 6,7 %)
11,5 Mg i. Tr. (Theorie 12,0 %)
41,4 C i. Tr. (Theorie 41,8 %)
Die Konzentration der Aluminium-Magnesium-Hydroxi-Verbindung in der neuen Gelzusammensetzung liegt zweckmäßigerweise bei 5 bis 25 Gew.%, bezogen auf die Gelzusammensetzung, vorzugsweise 10 bis 20 Gew.%.
Die organische bei Raumtemperatur flüssige Verbindung, in der die Gelbildung stattfindet, kann ausgewählt sein aus der Gruppe:
a) der pflanzlichen und tierischen Fette, Öle und Wachse
(z. B. Rizinusöl, Jojobaöl, Wollwachse),
b) der Paraffinkohlenwasserstoffe
(mit einem Siedebereich zwischen 170 - 550 C)
c) der Silikonöle
(z. B. Dimethicone, Cyclomethicone tetramer und pentamer),
d) der aliphatischen und aromatischen Ester
(z. B. Isopropylmyristat, Isopropylpalmitat, Di-Octyladipat)
e) der höheren Alkohole und Ether
(z. B. Polyethylenglykol, Octadodecanol).
Auch können Mischungen von Verbindungen aus einer Gruppe miteinander kombiniert werden, wenn sie miteinander kompatibel sind.
Die Konzentration der organischen lipophilen Verbindung sollte zwischen 95 und 75 Gew.%, bezogen auf die Gelzusammensetzung, liegen; vorzugsweise liegt sie zwischen 90 und 80 %.
Gemäß einer besonders günstigen Ausführungsweise enthalten die neuen Gelzusammensetzugen weiterhin ein polares Additiv in Mengen bis zu 20 Gew.%, bezogen auf die Aluminium-Magnesium-Hydroxiverbindung. In vielen Fällen reichen bereits Mengen von bis zu 1 Gew.% aus. Erwähnenswert ist an dieser Stelle, daß auf die Zugabe des Additivs sogar verzichtet werden kann.
Als polares Additiv können Gemische aus Wasser/Methanol und/oder Wasser/Ethanol verwendet werden. Auch sind Aceton, Propylencarbonat und Polyoxyethylen-(4)-laurylalkohol gut geeignet.

Die Herstellung der neuen Gelzusammensetzungen erfolgt derart, daß man die pulverförmige Aluminium-Magnesium-hydroxi-Verbindung, das polare Additiv und die lipophilen, bei Raumtemperatur flüssige Verbindung in einem geeigneten Mischer unter Anwendung hoher Scherkräfte auf 120 C bis 130 C erhitzt. Das dabei erhaltene Gel hat eine vaselinartige Konsistenz und ist transparent bis weiß. Sie ist für die Kosmetik ein optimales rheologisches Additiv und Antiabsetzmittel.

In den nachfolgenden Beispielen sind Herstellung und die physikalischen Eigenschaften der erfindungsgemäßen Gele, wie z.B. Viskosität, Farbe, Geruch, Stabilität, Thixotropie und Absetzverhalten ausführlich beschrieben.

Von hochviskosen Gelen wird die Konsistenz mit Hilfe der Mikropenetrationsmethode bestimmt. Die Messung erfolgte mit dem Penetrometer der Fa. Sommer & Runge in Berlin mit einem 5 g schweren Fallstab, der nach der Entarretierung 5 Sekunden lang in das zu messende Gel eindringt. Die Angabe der Eindringtiefe erfolgt in 0,1 mm-Angaben.

Die Abbildung 2 zeigt eine Abnahme der Viskosität der Gele mit Zunahme der Menge an polarem Additiv bis zu einem Minimum und anschließender Zunahme.

Beispiele 18-24: Herstellung von Mineralöl-Gelen mit verschiedenen polaren Additiven

In einem 400 ml Becherglas werden 20 g des Aluminium-Magnesium-hydroxi-stearat-Pulvers aus dem Beispiel 17 in Paraffinöl (Typ Pioneer 2 660, hochviscos, der Firma Hansen & Rosenthal in Hamburg) suspendiert und mit Hilfe einer Heizplatte unter Rühren auf 90 Grad C hochgeheizt. Dann gibt man das polare Additiv zu und erhitzt unter Rühren auf ca. 120 Grad C bis die Suspension leicht zu schäumen beginnt. Das Schäumen ist auf Spuren an Wasser zurückzuführen. Die Suspension wird nun ohne weiter zu kühlen ca. 30 Sekunden mit hohen Scherkräften (z. B. IKA-Labor-Turrax) behandelt bis sich eine Verdickung ausbildet. Danach wird die Suspension in einem Exsikator unter Vakuum und leichtem Rühren abgekühlt.

Es resultiert jeweils ein farbloses, transparentes Gelunterschiedlicher Viskosität.

Tabelle 5

| Herstellung von Mineralöl-Gelen mit verschiedenen polaren Additiven | | | | | | |
|---|---|---|---|---|---|---|
| Beispiel Nr. | Paraffinöl (g) | polares Additiv | | | Ausbeute | |
| | | Typ | (g) | (%)* | (g) | (%) |
| 18 | 180 | - | - | - | 200 | 100 |
| 19 | 180 | A | 0,1 | 0,5 | 199 | 99 |
| 20 | 179 | A | 1 | 5 | 199 | 99 |
| 21 | 178 | A | 2 | 10 | 197 | 98 |
| 22 | 176 | A | 4 | 20 | 195 | 97 |
| 23 | 179 | B | 1 | 5 | 199 | 99 |
| 24 | 178 | C | 1 | 5 | 198 | 99 |
| A = Polyoxyethylen-(4)-laurylalkohol | | | | | | |
| B = Ethanol/$H_2O$ 95/5 | | | | | | |
| C = Propylencarbonat | | | | | | |
| * bezogen auf das eingesetzte Pulver | | | | | | |

Beispiel 25-28: Herstellung von Mineralöl-Gelen mit Al-Mg-hydroxi-stearaten unterschiedlicher Zusammensetzung

In einem 400 ml Becherglas werden 30 g des Aluminium-Magnesium-hydroxi-stearat-Pulvers aus den Beispielen 12-15 in 170 g Paraffinöl (Typ Pioneer 2 660, hochviscos der Firma Hansen & Rosenthal in Hamburg) suspendiert und mit Hilfe einer Heizplatte unter Rühren auf 90 Grad C hochgeheizt. Dann gibt man 0,1 g Polyoxyethylen-(4)-laurylalkohol (0,5 % bezogen auf das eingesetzte Pulver) zu und erhitzt unter Rühren weiter auf ca. 120 Grad C bis die Suspension leicht zu schäumen beginnt. Das Schäumen ist auf Spuren an Wasser zurückzuführen. Die Suspension wird nun ohne weiter zu kühlen ca. 30 Sekunden mit hohen Scherkräften (z. B. IKA-Labor-Turrax) behandelt bis sich eine Verdickung ausbildet. Danach wird die

16

Suspension in einem Exsikkator unter Vakuum und leichtem Rühren abgekühlt.

Es resultieren transparente Gele unterschiedlicher Viscosität und Stabilität.

Tabelle 6

| Herstellung von Mineralöl-Gelen mit Al-Mg-hydroxistearaten unterschiedlicher Zusammensetzung | | | | | |
|---|---|---|---|---|---|
| Beispiel Nr. | Al-Mg-hydroxistearat aus Beispiel | Atomverhält. | | Ausbeute | |
| | | Al | Mg | g | % |
| 25 | 2 | 3 | 13 | 198 | 99 |
| 26 | 3 | 5 | 10 | 197 | 98 |
| 27 | 4 | 7 | 7 | 195 | 97 |
| 28 | 5 | 9 | 4 | 198 | 99 |

Beispiele 29-32: Herstellung von Mineralöl-Gelen mit unterschiedlichen Pulver-Konzentrationen

In einem 400 ml Becherglas werden Aluminium-Magnesium-hydroxi-stearat-Pulver aus Beispiel 16 in Paraffinöl (Typ Pioneer 2 660, hochviscos, der Firma Hansen & Rosenthal in Hamburg) suspendiert und mit Hilfe einer Heizplatte unter Rühren auf 90 Grad C hochgeheizt. Dann gibt man 0,1 g Polyoxyethylen-(4)-lauryalkohol (0,5 % bezogen auf das eingesetzte Pulver (zu und erhitzt unter Rühren weiter auf ca. 120 Grad C bis die Suspension leicht zu schäumen beginnt. Das Schäumen ist auf Spuren an Wasser zurückzuführen. Die Suspension wird nun ohne weiter zu kühlen ca. 30 Sekunden mit hohen Scherkräften (z. B. IKA-Labor-Turrax) behandelt bis sich eine Verdickung ausbildet. Danach wird die Suspension in einem Exsikator unter Vakuum und leichtem Rühren abgekühlt.

Es resultieren jeweils farblose, transparente Gel unterschiedlicher Viscosität.

Tabelle 7

| Herstellung von Mineralöl-Gelen mit unterschiedlicher Pulver-Konzentration | | | | | |
|---|---|---|---|---|---|
| Beispiel Nr. | Paraffinöl (g) | Al-Mg-hydroxi-stearat | | Ausbeute | |
| | | (g) | (%) | (g) | (%) |
| 29 | 190 | 10 | 5 | 198 | 99 |
| 30 | 180 | 20 | 10 | 199 | 100 |
| 31 | 174 | 26 | 13 | 200 | 100 |
| 32 | 160 | 40 | 20 | 199 | 99 |

Beispiele 33-40: Herstellung von Gelen mit unterschiedlichen organischen Flüssigkeiten

In einem 400 ml Becherglas werden 30 g des Aluminium-Magnesium-hydroxi-palmitat-stearat-Pulvers aus Beispiel 17 in 170 g einer organischen Flüssigkeit suspendiert und mit Hilfe einer Heizplatte unter Rühren auf 90 Grad C hochgeheizt. Dann gibt man 0,1 g Polyoxyethylen-(4)-laurylalkohol (0,5 % bezogen auf das eingesetzte Pulver) zu und erhitzt unter Rühren weiter auf ca. 120 Grad C bis die Suspension leicht zu schäumen beginnt. Das Schäumen ist auf Spuren an Wasser zurückzuführen. Die Suspension wird nun ohne weiter zu kühlen ca. 30 Sekunden mit hohen Scherkräften (z. B. IKA-Labor-Turrax) behandelt bis sich eine Verdickung ausbildet. Danach wird die Suspension in einem Exsikator unter Vakuum und leichtem Rühren abgekühlt.

Tabelle 8: Herstellung von Gelen mit unterschiedlichen organischen

EP 0 318 642 B1

```
                  Flüssigkeiten

       Beispiel Nr.            org. Flüssigkeit              Ausbeute
                                                             g      %
       -------------------------------------------------------------------
            33            Isopropylmyristat (1)            190      95
            34            Cyclomethicone(pentamere) (2)    194      97
            35            Cyclomethicone (tetramere) (2)   192      96
            36            Rizinusöl (3)                    190      95
            37            Jojobaöl (4)                     190      95
            38            2-Octyl-Dodecanol (1)            170      85
            39            Di-Octyl-Adipat (1)              190      95
            40            Lanolin (4)                      185      92
```

Beispiel 41: Herstellung von Gel mit Mineralöl

In einem Labormischer (Typ Unimix von Hagen & Rinau, Bremen) mit einem Maximalinhalt von 5 kg werden bei Raumtemperatur in 3 600 g Paraffinöl (Typ Pioneer 2 660, hochviscos der Firma Hansen & Rosenthal in Hamburg) 400 g Pulver aus Beispiel 17 suspendiert, 0,5 % Polyoxyethylen-(4)-lauryalkohol zugesetzt und unter leichtem Rühren auf 120 Grad C aufgeheizt. Die Heizung wird abgeschaltet und die Suspension mit hohen Scherkräften (Turrax) 10 Minuten behandelt. Dabei steigt die Temperatur auf ca. 130 Grad C an, die ca. 20 Minuten gehalten wird. Anschließend kühlt man unter Vakuum (ca. 0,6 bar) innerhalb von 2 Stunden auf Raumtemperatur ab.

Die Ausbeute an farblosem, transparentem Gel ist quantitiv.

Physikalische Eigenschaften

Die Versuche an den Beispielen 25 bis 28 zeigen, daß auch mit wechselnden Al : Mg-Verhältnissen Gele erhalten werden, die aber bei Abweichungen von dem Verhältnis 5 : 10 Instabilitäten aufweisen.

Tabelle 9

| Vergleich der Gele mit Mineralöl, hergestellt aus unterschiedlichen Al-Mg-Hydroxi-Stearat-Zusammensetzungen | | | | | |
|---|---|---|---|---|---|
| Gel aus | Aussehen | Mikropenetration 0,1 mm | Stabilitäten | | |
| | | | -18 Grad C 1 Tag nach dem Auftauen | 3 Monate bei Raum temperatur temperatur | 1 Woche bei 50 C |
| Beisp. 25 | farblos/transparent | 70 | A | B | B |
| Beisp. 26 | farblos/transparent | 108 | A | A | A |
| Beisp. 27 | weiß | 550 | A | D | D |
| Beisp. 28 | weiß | 209 | A | C | C |
| Beurteilung der Stabilität: A stabil B leichte Inhomogenität C beginnende Separation D deutliche Separation | | | | | |

Die nachstehende Tabelle 10 gibt eine Übersicht über die Zunahme (gemessen über die Mikropenetration bei 25 Grad C) der Gele aus den Beispielen 29 bis 32 mit unterschiedlichen Pulveranteilen. Die

18

EP 0 318 642 B1

Stabilität nimmt dabei mit zunehmendem Pulvergehalt zu.

Tabelle 10

| Vergleich der Gele mit unterschiedlichen Pulverkonzentrationen | | | | | |
|---|---|---|---|---|---|
| Gel aus | Pulverkonz.% | Mikropenetr. 0,1 mm | -18 Grad C 1 Tag nach Auftauen | 3 Mon. bei RT | 1 Woche 50 C |
| Beisp. 29 | 5 | 330 | A | A | B |
| Beisp. 30 | 10 | 124 | A | A | A |
| Beisp. 31 | 13 | 65 | A | A | A |
| Beisp. 32 | 20 | 42 | A | A | A |
| Beurteilung der Stabilität<br>A stabil<br>B leichte Inhomogenität<br>C beginnende Separation<br>D deutliche Separation | | | | | |

Um einen anschaulichen Vergleich der Konsistenz zu erhalten, wurde die Mikropenetration von Vaseline mit 103 (0,1 mm) bestimmt. Je niedriger der Wert, desto höher ist die Viskosität des Materials. Dieser Vergleich zeigt, daß die Gele aus den Beispielen der vorliegenden Anmeldung eine mit der Vaseline vergleichbare Konsistenz aufweisen.

Tabelle 11

| Vergleich der Gele aus den Beispielen mit Vaseline in Bezug auf die Konsistenz, gemessen mit der Mikropentrationsmethode bei 25 Grad C | | |
|---|---|---|
| Gel | Typ mit | Eindringtiefe (0,1 mm) |
| Beispiel 18 | Mineralöl | 104 |
| Beispiel 26 | Mineralöl | 55 |
| Beispiel 33 | IPM | 57 |
| Beispiel 34 | Cyclomethicone pentamer | 113 |
| Beispiel 35 | Cyclomethicone tetramer | 66 |
| Beispiel 36 | Rizinusöl | 64 |
| Beispiel 37 | Jojobaöl | 75 |
| Beispiel 38 | 2-Octyl-Dodecanol | 71 |
| Beispiel 39 | Di-Octyl-Adipat | 269 |
| Beispiel 40 | Lanolin | 34 |
| Vaseline | | 103 |

Ein wichtiges Merkmal von in der Kosmetik eingesetzten Gelen ist die Farbe. Zur Herstellung von absolut weißer Creme, z. B. Sonnenschutzmittel, sind weiße oder farblose Gele vorteilhaft. Die nachstehende Tab.12 zeigt einen Farbenvergleich mit im Handel erhältlichen Gelen. Hier zeigt sich die eindeutige Überlegenheit der erfindungsgemäßen Gele.

19

Tabelle 12

| Vergleich der Gele aus den Beispielen mit im Handel erhältlichen Gelen in Bezug auf Farbe | | |
|---|---|---|
| Gel | Typ mit | Farbe |
| Beispiel 18<br>Beispiel 26<br>Bentonit Gel<br>Miglyol Gel | Mineralöl<br>Mineralöl<br>Mineralöl<br>Neutralöl | farblos, transparent<br>farblos, transparent<br>braun, transparent<br>2 Qualitäten, grün und beige, nicht transparent |
| Beispiel 33<br>Bentonit Gel | IPM<br>IPM | weiß, nicht transparent<br>dunkelbeige, nicht transp. |
| Beispiel 34<br>Bentonit Gel | Cyclomethicone pentamer<br>Cyclomethicone pentamer | weiß, nicht transparent<br>beige, nicht transparent |
| Beispiel 35<br>Bentonit Gel | Cyclomethicone tetramer<br>Cyclomethicone tetramer | weiß, nicht transparent<br>beige, nicht transparent |
| Beispiel 36<br>Bentonit Gel | Rizinusöl<br>Rizinusöl | hellgelb, leicht transp.<br>dunkelbraun,leicht transp. |
| Beispiel 37<br>Beispiel 38<br>Beispiel 39<br>Beispiel 40 | Jojobaöl<br>2-Octyl-Dodecanol<br>Di-Octyl-Adipat<br>Lanolin | hellgelb, transparent<br>farblos bis weiß<br>beige<br>gelb |

In der Kosmetik werden die Formulierungen von Fertigprodukten mit zusätzlichen Schwierigkeiten bei der Parfümierung belastet, wenn die eingesetzten Produkte einen starken Eigengeruch aufweisen. Die untenstehende Tab. 13 zeigt einen Vergleich mit im Handel erhältlichen Produkten. Dabei zeigt sich auch wieder die Überlegenheit der durch die Erfindung beschriebenen Gele.

Tabelle 13

| Vergleich der Gele aus den Beispielen mit im Handel erhältlichen Gelen in Bezug auf Geruch | | |
|---|---|---|
| Gel | Typ mit | Geruch |
| Beispiel 18<br>Beispiel 26<br>Bentonit Gel<br>Miglyol Gel<br>Miglyol Gel | Mineralöl<br>Mineralöl<br>Mineralöl<br>Neutralöl<br>Neutralöl | geruchlos<br>geruchlos<br>Geruch nach Propylencarbonat<br>grüne Qualität, Eigengeruch<br>beige Qualität, geruchlos |
| Beispiel 33<br>Bentonit Gel | IPM<br>IPM | geruchlos<br>geruchlos |
| Beispiel 34<br>Bentonit Gel | Cyclomethicone pentamer<br>Cyclomethicone pentamer | geruchlos<br>starker Geruch nach Ethanol |
| Beispiel 35<br>Bentonit Gel | Cyclomethicone tetramer<br>Cyclomethicone tetramer | geruchlos<br>starker Geruch nach Ethanol |
| Beispiel 36<br>Bentonit Gel | Rizinusöl<br>Rizinusöl | Eigengeruch nach Rizinusöl<br>Eigengeruch nach Rizinusöl |
| Beispiel 37<br>Beispiel 38<br>Beispiel 39<br>Beispiel 40 | Jojobaöl<br>2-Octyl-Dodecanol<br>Di-Octyl-Adipat<br>Lanolin | leichter Eigengeruch nach Jojobaöl<br>leichter Eigengeruch<br>leichter Eigengeruch<br>Geruch nach Lanolin |

Ein Vergleich der Stabilitäten zeigt, daß die in der Erfindung beschriebenen Gele stabiler als im Handel erhältlichen Gele sind.

Tabelle 14

| Vergleich der Gele aus den Beispielen mit im Handel erhältlichen Gele in Bezug auf Stabilität | | | | |
|---|---|---|---|---|
| Gel | Typ mit | -18 Grad C 24 h aufgetaut | Raum-temp. 3 Monate | 50 Crad 7 Tage |
| Beispiel 18 | Mineralöl | A | A | A |
| Beispiel 26 | Mineralöl | A | A | A |
| Bentonit Gel | Mineralöl | A | B | A |
| Miglyol Gel grün | Neutralöl | A | D | C |
| Miglyol Gel beige | Neutralöl | A | B | B |
| Beispiel 33 | IPM | B | A | A |
| Bentonit Gel | IPM | B | A | A |
| Beispiel 34 | Cyclomethicone pentamer | A | A | C |
| Bentonit Gel | Cyclomethicone pentamer | A | F | C |
| Beispiel 35 | Cyclomethicone tetramer | A | A | A |
| Bentonit Gel | Cyclomethicone tetramer | A | F | A |
| Beispiel 36 | Rizinusöl | A | A | A |
| Bentonit Gel | Rizinusöl | A | A | A |
| Beispiel 37 | Jojobaöl | A | A | A |
| Beispiel 38 | 2-Octyl-Dodecanol | A | A | B |
| Beispiel 39 | Di-Octyl-Adipat | C | B | D |
| Beispiel 40 | Lanolin | A | A | A |
| Beurteilung: <br><br> A stabil <br><br> B leichte Inhomogenität <br><br> C beginnende Separation <br><br> D deutliche Separation <br><br> E totale Separation <br><br> F Verdunstung von Alkohol | | | | |

Eine wichtige Eigenschaft von quellfähigen Bentoniten ist das ausgesprochen thixotrope und verdickende Verhalten.

Die untenstehende Tab. 15 zeigt die Viskosität als Funktion der Schergeschwindigkeit in Abhängigkeit von der Temperatur am Gel aus Beispiel 41 gemessen mit dem Rheomat 115 der Firma Contraves in Stuttgart (Meßkörper DIN 125):

Tabelle 15:
Viskosität als Funktion der Schergeschwindigkeit in Abhängigkeit von der Temperatur

Schergeschw.*  Viskosität (mpa.s) des Gels aus Beispiel 41 bei T(Grad)

| $D$ (S$^{-1}$) | 20 Grad C | 30 Grad C | 40 Grad C | 50 Grad C | 60 Grad C |
|---|---|---|---|---|---|
| 6,65 | 17 158 | 10 811 | 7 207 | 4 462 | 3 089 |
| 3,51 | 12 120 | 7 680 | 5 040 | 3 120 | 2 280 |
| 12,61 | 8 631 | 5 447 | 3 687 | 2 263 | 1 676 |
| 10,48 | 6 387 | 3 985 | 2 637 | 1 641 | 1 231 |
| 27,9 | 4 826 | 2 863 | 1 881 | 1 186 | 941 |
| 39,9 | 3 775 | 2 145 | 1 401 | 887 | 686 |
| 57,2 | 2 993 | 1 616 | 1 037 | 658 | 519 |
| 81,8 | 2 441 | 1 270 | 809 | 502 | 405 |
| 117,1 | 2 016 | 1 013 | 643 | 409 | 321 |
| 167,6 | 1 682 | 831 | 518 | 327 | 259 |
| 240 | 1 420 | 689 | 428 | 276 | 214 |
| 343 | 1 209 | 596 | 366 | 233 | 180 |
| 492 | 1 030 | 524 | 318 | 202 | 153 |
| 704 | 878 | 465 | 277 | 177 | 132 |
| 1008 | 758 | 421 | 252 | 162 | 120 |

* Die Änderung der Schergeschwindigkeit erfolgte in 15 Stufen nach jeweils 30 Sekunden

Aus der Tabelle 15 ist die Abnahme der Viskosität mit der Zunahme der Schergeschwindigkeit und der Temperatur sehr gut zu erkennen. Die nachfolgende Tabelle 16 zeigt die Viskosität als Funktion der Zunahme und anschließender Abnahme der Schergeschwindigkeit des Gels aus Beispiel 26 bei unterschiedlichen Temperaturen. Aus der Tabelle 16 ist deutlich das thixotrope Verhalten durch die unterschiedlichen Viskositäten im Anfangs- und Endwert zu erkennen.

EP 0 318 642 B1

Tabelle 16

| Viskosität als Funktion der Schergeschwindigkeit in Abhängigkeit von der Temperatur | | | | |
|---|---|---|---|---|
| Schergeschwindigkeit* D (S ) | Viskosität (mpa.s) bei T (Grad C) | | | |
| | 20 Grad C | | 80 Grad C | |
| 6,65 | 25 528 | 14 414 | 9 953 | 6 349 |
| 9,51 | 18 600 | 10 080 | 6 840 | 4 680 |
| 13,61 | 13 408 | 8 212 | 4 777 | 3 268 |
| 19,48 | 9 786 | 6 211 | 3 340 | 2 403 |
| 27,9 | 7 198 | 4 785 | 2 372 | 1 759 |
| 39,9 | 5 320 | 3 804 | 1 687 | 1 287 |
| 57,2 | 4 070 | 3 092 | 1 177 | 938 |
| 81,8 | 3 139 | 2 595 | 865 | 712 |
| 117,1 | 2 503 | 2 153 | 633 | 536 |
| 167,6 | 2 016 | 1 798 | 470 | 409 |
| 240 | 1 672 | 1 501 | 347 | 312 |
| 343 | 1 399 | 1 299 | 273 | 250 |
| 492 | 1 199 | 1 200 | 209 | 200 |
| 704 | 1 034 | 956 | 169 | 164 |
| 1008 | 872 | 872 | 137 | 137 |

* Die Änderung der Schergeschwindigkeit erfolgte in 15 Stufen nach jeweils 2 Minuten ansteigend und abfallend.

Wie eingangs schon beschrieben, sind die thixotropen Eigenschaften von Gelen für die Kosmetik äußerst wichtig. Einen Vergleich mit im Handel erhältlichen Gelen zeigt die untenstehende Tabelle 17. Es wurde die Viskosität in Abhängigkeit von der Zu- und Abnahme der Schergeschwindigkeit bei 20 Grad C von dem Gel aus Beispiel 31 und dem Bentonit Gel mit Mineralöl gemessen.

Tabelle 17

| Viskosität als Funktion der Zu- und Abnahme der Schergeschwindigkeit bei 20 Grad C | | | | |
|---|---|---|---|---|
| Schergeschwindigkeit* D (S ) | Gel aus Beispiel 31 Viskosität (mpa.s) | | Bentonit Gel Viskosität (mpa.s) | |
| 6,65 | 20 935 | 12 184 | 123 895 | 115 315 |
| 9,51 | 14 640 | 8 760 | 87 480 | 84 600 |
| 13,61 | 10 391 | 6 453 | 62 431 | 62 012 |
| 19,48 | 7 500 | 4 964 | 45 884 | 45 532 |
| 27,9 | 5 440 | 3 681 | 33 333 | 33 374 |
| 39,9 | 4 061 | 2 860 | 24 367 | 24 567 |
| 57,2 | 3 092 | 2 274 | 17 835 | 17 995 |
| 81,8 | 2 427 | 1 841 | 13 085 | 13 141 |
| 117,1 | 1 968 | 1 519 | 9 545 | 9 516 |
| 167,6 | 1 614 | 1 280 | 6 946 | 6 871 |
| 240 | 1 340 | 1 026 | 5 016 | 4 950 |
| 343 | 1 132 | 952 | 3 663 | 3 730 |
| 492 | 958 | 840 | 2 768 | 2 598 |
| 704 | 807 | 747 | 1 921 | 1 887 |
| 1008 | 687 | 687 | 1 407 | 1 407 |

* Die Änderung der Schergeschwindigkeit erfolgte in 15 Stufen nach jeweils 2 Minuten ansteigend und abfallend.

Beim Vergleich der beiden Gelqualitäten miteinander sieht man bei dem erfindungsgemäßen Gel ein höheres thixotropes Verhalten, welches sich durch die größere Differenz des Anfangs- und Endwertes zeigt.

23

Verwendung der Gelzusammensetzung in kosmetischen Zusammensetzungen

Um die Vorteile in der Anwendung der durch die Erfindung beschriebenen Gele gegenüber herkömmlichen Gelen aufzuzeigen, wurden mehrere Fertigformulierungen hergestellt und getestet.

1.Herstellung von thermostabilen W/O-Sonnencremes:

Ein Problem bei der Herstellung von Sonnencreme, welches bis heute noch nicht optimal gelöst ist, ist die Instabilität der Viskosität von Cremen bei höheren Temperaturen bis 60 Grad C, wie sie in der Sonne am Strand oder in Auslagen vorkommen können. Bei diesen Temperaturen ist eine herkömmliche Creme flüssig und läßt sich nicht mehr gut auftragen. Sie läuft wie Wasser weg. Wenn das in dem Beispiel 18 hergestellte Gel in einer Konzentration bis zu 15 % in eine Creme eingearbeitet wird, ist die Viskosität bei 60 Grad C noch so hoch, daß sich die Creme gut auftragen läßt. Außerdem wird die Langzeitstabilität start verbessert.

Zum Vergleich wurden untenstehende Creme-Rezepturen getestet:

Tabelle 18

| Formulierung (in %) | A | B | C | D | E | F |
|---|---|---|---|---|---|---|
| Gel aus Beispiel 18 | 15 | - | - | - | - | - |
| Bentonit Gel mit Mineralöl (1) | - | 15 | - | - | - | - |
| Miglyol-Gel (2) | - | - | 15 | - | - | - |
| Aluminiumstearat (3) | - | - | - | 2,3 | - | - |
| Magnesiumstearat (3) | - | - | - | - | - | 2,3 |
| Paraffinöl (4) | 8,5 | 8,5 | 8,5 | 21,2 | 23,5 | 21,2 |
| Cetylalkohol (5) | 2 | 2 | 2 | 2 | 2 | 2 |
| Sorbitan-Monoisostearat (6) | 3 | 3 | 3 | 3 | 3 | 3 |
| Lanolin (7) | 1 | 1 | 1 | 1 | 1 | 1 |
| Mikrowachs (HP 67)(8) | 6 | 6 | 6 | 6 | 6 | 6 |
| Wasser | 63,8 | 63,8 | 63,8 | 63,8 | 63,8 | 63,8 |
| Phenonip (Konserv.) (9) | 0,2 | 0,2 | 0,2 | 0,2 | 0,2 | 0,2 |
| Parfümöl | 0,5 | 0,5 | 0,5 | 0,5 | 0,5 | 0,5 |

Produkte der Hersteller:
(1) NL Chemicals
(2) Dynamit Nobel, Troisdorf
(3) Bärlocher,München
(4) Hansen & Rosenthal, Bremen
(6) Henkel, Düsseldorf
(7) Nordmann & Rassmann, Hamburg
(8) Schütz, Oberursel
(9) Damm, Hamburg

Die Herstellung der Creme erfolgt durch Aufschmelzen der Ölphase bei 80 Grad C und Zusatz des 80 Grad C heißen Wassers unter Rühren. Man rührt ca. 5 Minuten und kühlt dann auf ca. 35 Grad C ab. Anschließend wird das Parfümöl und das Konservierungsmittel zugefügt. Die Konsistenz der Cremes wird wieder über die Mikropenetration bestimmt. Aus der beiliegenden Abb. 3 ist ersichtlich, daß die üblicherweise auf der Basis von Kohlenwasserstoff hergestellten Cremes bei über 40 Grad C flüssig werden (gestrichelte Linie), während die Formulierung A mit dem Gel aus Beispiel 18 noch bei Temperaturen über 70 Grad C eine halbfeste Konsistenz aufweist. Ein weiteres Problem bei der Herstellung von Suspensionen in der Kosmetik besteht in der schnellen Sedimentation de unlöslichen Anteile. Zum Beispiel bei Antiperspirant-Aerosole, die aus Treibgas, Siliconöl und dem unlöslichen aktiven Wirkstoff Aluminiumchlorohydrat bestehen, sollte der Wirkstoff nach dem Aufschütteln noch lange homogen in der Suspension verteilt bleiben. Ist dies nicht der Fall, würden sich bei der Anwendung je nach Dauer unterschiedliche Wirkstoffkonzentrationen ergeben. Auch bei den Suspensions-Roll-ons tritt das gleiche Problem auf. Geradezu in idealer Weise für den obigen Einsatz sind die Gele aus den Beispielen 33 bis 35 geeignet.

Für einen Vergleich mit und ohne rheologischen Additiv wurden folgende Aerosol-Formulierungen angefertigt und das Absetzverhalten überprüft. Hierzu wird das Absetzvolumen nach verschiedenen Zeiten bestimmt. Die Aerosol-Glasflasche wird 20mal vertikal und 20mal horizontal geschüttelt und dann stehen gelassen. Das Gesamtvolumen der Suspension beträgt 100 %. Das Absetzvolumen ist das Gesamtvolumen minus des überstehenden Treibgases nach den entsprechenden Zeiten, angegeben in %.

Tabelle 19

| Aerosol-Formulierungen (in %) | A | B | C |
|---|---|---|---|
| Gel aus Beispiel 35 | 5 | - | - |
| Bentonit Gel (tetramer) | - | 5 | - |
| Cyclomethicone (tetramer) | 3 | 3 | 8 |
| Isopropylmyristat | 2 | 2 | 2 |
| Ethanol | 2 | 2 | 2 |
| Aluminiumchlorohydrat | 3 | 3 | 3 |
| Parfümöl | 0,5 | 0,5 | 0,5 |
| Butan | 84,5 | 84,5 | 84,5 |

Herstellung:

Die Produkte, mit Ausnahme des Treibmittels, werden homogen vermischt, in Spraydosen abgefüllt und mit dem Kohlenwasserstofftreibgas versetzt.

Tabelle 20

| Suspensions-Roll-on-Formulierungen | D | E | F |
|---|---|---|---|
| Gel aus Beispiel 34 | 10 | - | - |
| Bentonit Gel mit Cyclomethicone (pentamer) | - | 10 | - |
| Cyclomethicone (pentamer) | 64,5 | 64,5 | 64,5 |
| Isopropylmyristat | 2 | 2 | 2 |
| Ethanol | 3 | 3 | 3 |
| Aluminiumchlorohydrat | 20 | 20 | 20 |
| Parfümöl | 0,5 | 0,5 | 0,5 |

Herstellung:

In das Cyclomethicone (pentamer) werden in der nachfolgenden Reihenfolge die Komponenten Gel, Isopropylmyristat, Ethanol, Aluminiumchlorohydrat, Parfümöl homogen eingerührt. In den beistehenden Abb. 4 und 5 sind die Absetzkurven für die Formulierungen aufgezeichnet. Deutlich ist der Unterschied zu Formulierung ohne rheologisches Additiv und damit der Vorteil in der Erfindung beschriebenen Gele zu erkennen. Ein weiteres Problem der Fertigkosmetika besteht speziell bei Lippenstiften und Lip-Gloss-Präparaten in wechselnden Lagertemperaturen, z.B. Nacht/ Tag und der Empfindlichkeit der Konsistenz gegenüber Temperaturen über 40 Grad C, z.B. in Sommer. Außerdem sedimentieren die Farbstoffpigmente im geschmolzenen Zustand bei der Herstellung.

Zur Untersuchung der obigen Parameter wurden die untenstehenden Formulierungen im Labor herge-stellt:

Tabelle 21:

| Formulierungen (in %) | | A | B | C |
|---|---|---|---|---|
| Gel aus Beispiel 33 | | - | 10 | - |
| Gel aus Beispiel 36 | | - | - | 10 |
| Rizinusöl | (1) | 51,2 | 50,2 | 41,2 |
| Isopropylmyristat | (2) | 10 | 1 | 10 |
| Ozekerit | (3) | 5 | 5 | 5 |
| Candellilla Wachs | (3) | 9 | 9 | 9 |
| Amerchol L 101 | (4) | 2,5 | 2,5 | 2,5 |
| Pigmentanreibung* | | 20 | 20 | 20 |
| BHT | (1) | 0,1 | 0,1 | 0,1 |
| Propylparaben | (1) | 0,1 | 0,1 | 0,1 |
| Parfümöl | | 0,5 | 0,5 | 0,5 |

* Pigmentanreibung: 80 % von 1 und 20 % von 2

1 = 50 % Titandioxid in Rizinusöl(5)
2 = 3 % Erytrosinlack (Rot) in Rizinusöl (5)
Produkte der Hersteller:
(1) Merck, Darmstadt
(2) Henkel, Düsseldorf
(3) Schütz, Oberursel
(4) Nordmann & Rassmann, Hamburg
(5) BASF

Herstellung der Lippenstifte nach den Formulierungen A, B, C gemäß Tabelle 21:

Die Komponenten (außer der Pigmentanreibung, BHT, Propylparaben und Parfümöl) werden bei 90 Grad C unter Rühren aufgeschmolzen, danach auf ca. 70 Grad C unter Vakuum abgekühlt und die Pigmentanreibung hinzugegeben. Anschließend fügt man die restlichen Komponenten hinzu und füllt ab.

Zur Untersuchung der wechselnden Lagertemperaturen wurden die obigen Formulierungen eine Nacht bei + 5 Grad C und anschließend 8 Stunden bei 40 Grad C gelagert. Der visuelle Vergleich der Proben zeigt deutlich ein Ausölen der Formulierung A, während die Formulierungen B und C einwandfrei waren. Zur Beobachtung einer möglichen Sedimentation von Farbpigmenten im geschmolzenen Zustand wurden die erneut geschmolzenen Formulierungen A bis C bei 80 Grad C in 80 Grad C heiße Glasrohre mit 1 cm Innendurchmesser und 10 cm Länge gefüllt und bei Raumtemperatur abkühlen gelassen. Hier zeigt sich ein leichter Unterschied in der Farbintensität des oberen zu dem unteren Teil bei der Formulierung A, während bei den Formulierungen B und C kein Unterschied sichtbar war.

Herstellung von stabilen Wasser in Silicon-Formulierungen:

Ein bis heute in der Kosmetik noch nicht befriedigen gelöstes Problem ist die Herstellung von stabilen Wasser in Siliconöl-Formulierungen. Meistens ölen die Produkte schon nach kurzer Zeit aus oder sind bei höheren Temperaturen sofort instabil. Mit den erfindungsgemäßen Gelen kann, wie Tabelle 22 zeigt, auch dieses Problem gelöst werden. Verglichen werden die Stabilitäten einer Creme, hergestellt aus dem Gel des Beispiels 34, und einer Creme aus pentameren Cyclomethicone.

Die Herstellung der Creme erfolgt durch Aufschmelzen der Ölphase bei 80 Grad C und Zusatz des 80 Grad C heißen Wassers unter Rühren. Danach kühlt man ab.

8688888888888888888 EP 0 318 642 B1

**Tabelle 22:**

| Formulierungen | A | B |
|---|---|---|
| Gel aus Beispiel 34 | 20 | - |
| Pentameres Cyclomethicone (1) | - | 20 |
| Emulgator O 2 - 3225 C    (1) | 10 | 10 |
| Bienenwachs | 6 | 6 |
| Emulgator Witconol 14    (2) | 2 | 2 |
| Wasser | 62 | 62 |

(1) Dow Corning

(2) Witco Chemicals

Der Vergleich der Stabilitäten wurde durch Lagerung bei Raumtemperatur und 1 Woche bei 60 Grad C durchgeführt. Die Formulierung B zeigt leichte Inhomogenitäten nach 4 Wochen Lagerung bei Raumtemprratur und deutliche Separation bei 60 Grad C, während die Formulierung A mit dem Gel aus Beispiel 34 absolut stabil war.

**Patentansprüche**

1. Gelzusammensetzung, enthaltend eine Aluminium-Magnesium-Hydroxi-Verbindung mit Schichtstruktur der allgemeinen Formel:

$$Al_xMg_y(OH)_{35-z}R_z \, n \, H_2O$$

in der R den Rest einer Monocarbonsäure $RCOO^-$ darstellt, und $RCOO^-$ 2 bis 22 Kohlenstoffatome enthält und die Indices x, y und z folgende Bedingungen erfüllen:

$3 \leq x \leq 9$
$4 \leq y \leq 13$
$3 \leq z \leq 5$, und $3x + 2y = 35$,

sowie eine bei Raumtemperatur (20 Grad C) flüssige, organische, lipophile Verbindung.

2. Gelzusammensetzung nach Anspruch 1, dadurch gekennzeichnet, daß die organische, lipophile Verbindung mindestens eine Verbindung aus der Gruppe
    a) der pflanzlichen und tierischen Fette, Öle und Wachse,
    b) der Paraffinkohlenwasserstoffe,
    c) der Silikonöle,
    d) der aliphatischen und aromatischen Ester oder
    e) der höheren Alkohole und Ether, ist.

3. Gelzusammensetzung nach den Ansprüchen 1 und 2, dadurch gekennzeichnet, daß sie bis 20 Gew.%, vorzugsweise bis 1 Gew.% eines polaren Additivs, bezogen auf die Aluminium-Magnesium-Hydroxi-

27

Verbindung, enthält.

**4.** Gelzusammensetzung nach den Ansprüchen 1 bis 3, dadurch gekennzeichnet, daß das polare Additiv ein Gemisch aus Wasser/Methanol, Wasser/Ethanol oder Aceton, Propylencarbonat oder Polyoxyethylen-(4)-laurylalkohol ist.

**5.** Gelzusammensetzung nach den Ansprüchen 1 bis 4, dadurch gekennzeichnet, daß die Konzentration der organischen, lipophilen Verbindung 95 bis 75 Gew.%, vorzugsweise 90 bis 80 Gew.% beträgt.

**6.** Gelzusammensetzung nach Ansprüchen 1 bis 5, dadurch gekennzeichnet, daß die Konzentration der Aluminium-Magnesium-Hydroxi-Verbindung 5 bis 25 Gew.%, vorzugsweise 10 bis 20 Gew.%, bezogen auf die Gelzusammensetzung, beträgt.

**7.** Verfahren zur Herstellung der Gelzusammensetzung nach Anspruch 1, dadurch gekennzeichnet, daß man die Komponenten unter Einwirkung von Scherkräften auf eine Temperatur von 120 bis 130 Grad C erhitzt.

**8.** Verwendung der neuen Gelzusammensetzung nach den Ansprüchen 1 bis 6 in kosmetischen Zusammensetzungen.

**Claims**

**1.** Gel composition containing an aluminium-magnesium-hydroxy compound with a layered structure of the general formula

$$Al_xMg_y(OH)_{35-z}R_z \; n \; H_2O$$

wherein R denotes the group of a monocarboxylic acid $RCOO^-$ and $RCOO^-$ contains 2 to 22 carbon atoms and the indices x, y and z satisfy the following conditions:

$3 \leq x \leq 9$
$4 \leq y \leq 13$
$3 \leq z \leq 5$, and $3x + 2y = 35$,

and an organic, lipophilic compound which is liquid at ambient temperature (20°C).

**2.** Gel composition according to claim 1, characterised in that the organic, lipophilic compound is at least one compound selected from the group
   a) of the vegetable and animal fats, oils and waxes,
   b) the paraffin hydrocarbons,
   c) the silicon oils,
   d) the aliphatic and aromatic esters or
   e) the higher alcohols and ethers.

**3.** Gel composition according to claims 1 and 2, characterised in that it contains up to 20 wt.%, preferably up to 1 wt.% of a polar additive, based on the aluminium-magnesium-hydroxy compound.

**4.** Gel composition according to claims 1 to 3, characterised in that the polar additive is a mixture of water/methanol, water/ethanol or acetone, propylene carbonate or polyoxyethylene-(4)-lauryl alcohol.

**5.** Gel composition according to claims 1 to 4, characterised in that the concentration of the organic, lipophilic compound is 95 to 75 wt.%, preferably 90 to 80 wt.%.

**6.** Gel composition according to claims 1 to 5, characterised in that the concentration of the aluminium-magnesium-hydroxy compound is 5 to 25 wt.%, preferably 10 to 20 wt.%, based on the gel composition.

7. Process for preparing the gel composition according to claim 1, characterised in that the components are heated to a temperature of 120 to 130°C under the action of shear forces.

8. Use of the new gel composition according to claims 1 to 6 in cosmetic preparations.

**Revendications**

1. Composition de gel, contenant un composé hydroxy d'aluminium et de magnésium présentant une structure stratifiée, répondant à la formule générale

$$Al_xMg_y(OH)_{35-z}R_z \ n \ H_2O$$

dans laquelle R signifie le reste d'un acide monocarboxylique $RCOO^-$ et contient 2 à 22 atomes de carbone, et les indices x, y et z répondent aux conditions suivantes:

$3 \leq x \leq 9$
$4 \leq y \leq 13$
$3 \leq z \leq 5$, et $3x + 2y = 35$,

ainsi qu'un composé organique lipophile solide à température ambiante (20°C).

2. Composition de gel selon la revendication 1, caractérisée en ce que le composé organique lipophile signifie au moins une substance appartenant à l'un des groupes suivants:
   a) les graisses, huiles et cires végétales et animales,
   b) les hydrocarbures paraffiniques,
   c) les huiles de silicone,
   d) les esters aliphatiques et aromatiques,
   e) les éthers et les alcools supérieurs.

3. Composition de gel selon les revendications 1 et 2, caractérisée en ce qu'elle contient 20%, de préférence jusqu'à 1% en poids, par rapport au composé hydroxy d'aluminium/magnésium, d'un additif polaire.

4. Composition de gel selon les revendications 1 à 3, caractérisée en ce que l'additif polaire est un mélange eau/méthanol, eau/éthanol ou de l'acétone, du carbonate de propylène ou l'alcool polyoxyéthylène-(4)-laurylique

5. Composition de gel selon les revendications 1 à 4, caractérisée en ce que la concentration des composés organiques lipophiles est de 95 à 75% en poids, de préférence 90 à 80% en poids.

6. Composition de gel selon les revendications 1 à 5, caractérisée en ce que la concentration du composé hydroxy d'aluminium et de magnésium est de 5 à 25% en poids, de préférence 10 à 20% en poids, par rapport à la composition de gel.

7. Procédé pour la fabrication de la composition de gel selon la revendication 1, caractérisé en ce que l'on chauffe les composants sous l'action de forces de cisaillement jusqu'à une température de 120 à 130°C.

8. Utilisation de la nouvelle composition de gel selon les revendications 1 à 6 dans des préparations cosmétiques.